# EUROPEAN PATENT SPECIFICATION

(11) **EP 1 835 949 B1**
(45) Date of publication and mention of the grant of the patent: **20.10.2010**
(21) Application number: 05854448.7
(22) Date of filing: 15.12.2005
(51) Int. Cl.: A61L 27/38, A61L 27/18

(54) **TISSUE ENGINEERING DEVICES FOR THE REPAIR AND REGENERATION OF TISSUE**
GEWEBEKONSTRUKTIONSVORRICHTUNGEN ZUR REPARATUR UND REGENERATION VON GEWEBE
DISPOSITIFS D'INGENIERIE TISSULAIRE DESTINES A REPARER ET A REGENERER DES TISSUS

(30) Priority: 21.12.2004 US 637984 P
(43) Date of publication of application: 26.09.2007
(73) Proprietor: ETHICON, INC., Somerville, NJ 08876-1515 (US)
(72) Inventor: SEYDA, Agnieszka, New Brunswick, New Jersey 08901 (US); SCOPELIANOS, Angelo G., Hamilton, New Jersey 08610 (US); GEESIN, Jeffrey C., North Brunswick, New Jersey 08902 (US); GOSIEWSKA, Anna, Raritan, New Jersey 08869 (US); SRIDEVI, Dhanaraj, Skillman, New Jersey 08558 (US); BUENSUCESO, Charito S., Doylestown, Pennsylvania 18901 (US); COLTER, David C., Whitehouse Station, New Jersey 08889 (US)
(74) Representative: Mercer, Christopher Paul
(86) International application number: PCT/US2005/045732
(87) International publication number: WO 2006/068972

(56) References cited:
- EP-A- 1 405 649
- EP-A- 1 464 346
- EP-A1- 1 216 718
- US-B1- 6 511 511

## Description

### FIELD OF THE INVENTION

The present invention relates to a method of making tissue engineering devices for use in the repair or regeneration of tissue. Specifically, this invention relates to a method ot making tissue engineering devices made of support scaffolds and cell sheets.

### BACKGROUND OF THE INVENTION

Considerable attention and effort has recently been directed to the substitution of defective organs with operationally effective replacements. The replacements have ranged from completely synthetic devices, such as artificial hearts, to completely natural organs from another mammalian donor. The field of heart transplants has been especially successful with the use of both synthetic hearts and natural hearts from living donors. Equal success has not been achieved in many other organ fields.

The recent emergence of tissue engineering and regenerative medicine offers alternative approaches to repair and regenerate/restore damaged or diseased tissue form and function. Tissue engineering strategies have explored the use of biomaterials in combination with cells and/or growth factors to develop biological substitutes that ultimately can restore or improve tissue function. The use of colonizable and remodelable scaffolding materials has been studied extensively as tissue templates, conduits, barriers and reservoirs. In particular, synthetic and natural materials in the form of foams, sponges, gels, hydrogels, textiles and non-wovens have been used *in vitro* and *in vivo* to reconstruct/regenerate biological tissue, as well as deliver chemotactic agents for inducing tissue growth. In some cases, cells are loosely seeded onto the scaffolding materials and immediately implanted into the body. Alternatively, cells may be loosely seeded onto the scaffolding materials and cultured for a period of time prior to implantation.

Methods for loosely seeding cells onto scaffolding materials are described in EP-A-1 405 649, EP-A-1 216 718 and US-B1-6 511 511.

Loosely seeding cells, however, lacks the ability to control the distribution of cells on the surface of the scaffold, especially if a uniform distribution is required. What is needed is a method of making a device in which the distribution of cells on the surface of the scaffold is controllable. Furthermore, viability of loosely seeded cells might be compromised since cells require supportive factors, such as extracellular matrix proteins, for attachment, proliferation and migration.

### SUMMARY OF THE INVENTION

The present invention is a method of making a tissue engineering device for use in the repair or regeneration of tissue having a support scaffold layer and a cell sheet layer, provided that the device does not comprise human embryonic stem cells. The method is defined in claim 1. Preferred features are defined in claims 2 to 9.

### BRIEF DESCRIPTION OF THE FIGURES

Fig. 1 is a schematic drawing of one embodiment of a device made according to the present invention.
Fig. 2 is an outline of a method of the present invention of making the tissue engineering device.

### DETAILED DESCRIPTION OF THE INVENTION

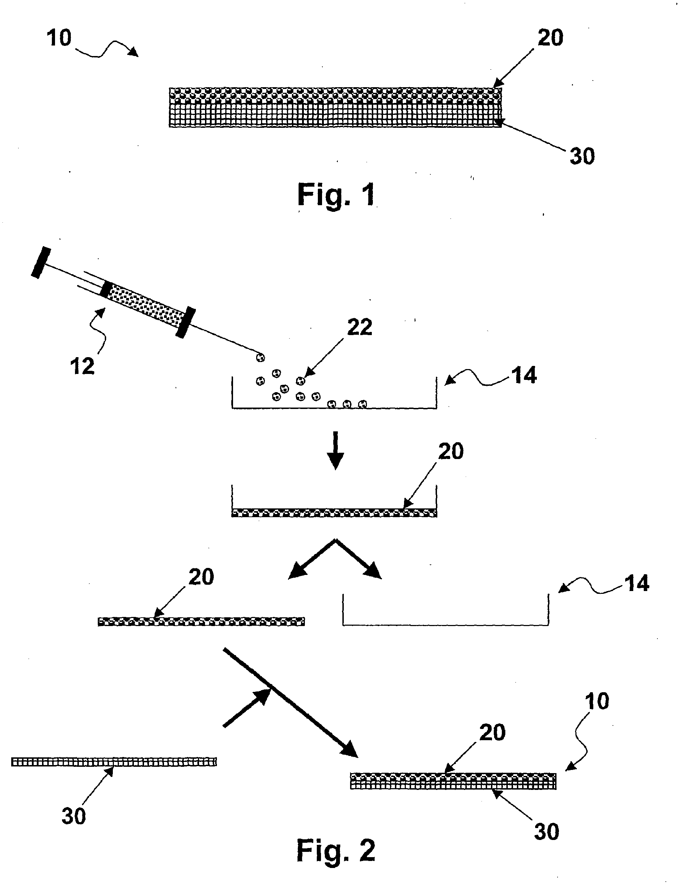

Fig. 1 is a schematic drawing showing a tissue engineering device **10** comprised of cell sheet layer **20** and scaffold layer **30.**

A method of the present invention of making a tissue engineering device **10** is shown in Fig 2. Selected cells **22** are seeded on a cell growth support device **14,** such as a Petri dish, by use of an inoculation device **12,** typically a syringe or a pipette. Cells **22** are grown to over 85% confluence, preferably about 100% confluence, on cell growth support device **14.** The over 85% confluent cells form cell sheet layer **20** on cell growth support device **14**. Cell sheet layer **20** is then separated from cell growth support device **14** and disposed on scaffold layer **30** to create tissue engineering device **10.**

Selected cells **22** may be autologous or may be derived allogeneically or xenogeneically.

Cells **22** may be obtained by treatment of an appropriate organ or tissue that is to serve as the source of the cells. Techniques for treatment of an organ or tissue to obtain stromal cells arc known to those skilled in the art. For example, the tissue or organ can be mechanically disrupted and/or treated with digestive enzymes or chelating agents to weaken the interactions between cells, making it possible to obtain a suspension of individual cells. Typically, the method will include a combination of mechanical disruption, enzyme treatment and treatment with chelating agents. In one technique, the tissue or organ is minced and treated simultaneously or subsequently with any of a number of digestive enzymes either alone or in combination. Examples of enzymes useful in dissociating cells include, but are not limited to, trypsin, chymotrypsin, collagenase, elastase, hyaluronidase, DNasc, pronase, dispase and the like. Mechanical disruption can also be accomplished by, for example, the use of blenders, sieves, homogenizers, pressure cells and the like.

The resulting suspension of cells and cell clusters can be further divided into population of substantially homogenous cell types. This can be accomplished using standard techniques for cell separation including, for example, positive selection methods (e.g. clonal expansion and selection of specific cell types), negative selection (e.g. lysis of unwanted cells), separation based upon specific gravity in a density solution, differential adherence properties of the cells in the mixed population, fluorescent activated cell sorting (FACS) and the like. Cells obtained by these methods are further expanded in culture using standard cell culture techniques known to those skilled in the art to obtain sufficient cell numbers as required for seeding on to the dishes,

Alternatively, cells may be obtained through a point-of-care approach. Where autologous cells are used, considerations of immunological response by the body are minimized or eliminated, while using allogeneic or xenogeneic cells may require further consideration of such immunological response by the body. Such a treatment strategy may require adjunct immunotherapy with cyclosporine, FK-506 or other such immunotherapeutic agents. Alternatively, cells might be transfected with genetic material which is capable of reducing or eliminating an immune response in the host.

Selected cells **22** that may be utilized according to the present invention include, but are not limited to, chondrocytes, osteoblasts, fibroblasts, angioblasts, myoblasts, epithelial cells, urothelial cells, smooth muscle cells, keratinocytes, beta cells, endothelial cells, fibrocytes, vascular endothelial cells, hepatocytes, small intestine epithelial cells, epidermal keratinocytes, bone marrow mesenchymal cells, cardiomyocytes, intervertebral disc cells, oral and gastrointestinal mucosal epithelial cells, urinary tract epithelial cells, as well as epithelial cells derived from other organ systems, skeletal joint synovium cells, periosteum cells, perichondrium cells, muscle cells (e.g. skeletal, smooth, cardiac), pericardium cells, dural cells, meningeals cells, keratinocyte-precursor cells, pericytes, glial cells, neural cells, amniotic and placental membranes cells, serosal cells (e.g., serosal cells lining body cavities) , undifferentiated or pre-differentiated stem or progenitor cells, neural stem or progenitor cells, neuronal cells, dendritic cells and genetically engineered cells. Stem cells include, without limitation, hematopoietic, mesenchymal, postpartum, pancreatic, hepatic, retinal epithelial, olfactory bulb, endothelial, muscle, adipose-derived, ilcac crest, bone marrow, periodontal ligament, oval and dermal stem cells and organ specific stem cells or progenitor cells, provided that they are not human embryonic stem cells.

Cells of any animal, including arthropods (insects), can be used, as well as germs and embryos can also be used, provided that they are not human embryonic stem cells. Examples include, non-human embryonic stem cells, somatic stem cell, neural stem cell, melanocytes, vascular smooth muscle cells, hair metrocytes, stellate cells, small hepatocytes, amnion-derived cells, fetal liver-derived cells, fetal kidney-derived cells, and fetal lung-derived cells, as well as cells of established cell lines such as HeLa cells, FL cells, KB cells, HepG2 cells, WI-88 cells, MA104 cells, BSC-1 cells, Vero cells, CV-1 cells, BHK-21 cells, L cells, CHL cells, BAE cells, BRL cells, PAE cells and the like.

In one embodiment, a genetically engineered cell sheet layer **20** is prepared. The genetically engineered cell sheet layer **20** comprises a population of cells **22** wherein at least one cell of the population of cells is transfected with an exogenous polynucleotide such that the exogenous polynucleotide expresses a diagnostic and/or therapeutic product (e. g. a polypeptide or polynucleotide) to assist in tissue healing, replacement, maintenance and diagnosis. Examples of "proteins of interest" (and the genes encoding same) that may be employed herein include, without limitation, cytokines, growth factors, chemokines, chemotactic peptides, tissue inhibitors of metalloproteinases, hormones, angiogenesis modulators (either stimulatory or inhibitory), immune modulatory proteins, neuroprotective and neuroregenerative proteins and apoptosis inhibitors. More specifically, preferred proteins include, without limitation, erythropoietin (EPO), EGF, VBGF, FGF, PDGF, IGF, KGF, IFN-α, IFN-δ, MSII, TGF-α, TGF-β, TNF-α, IL-1, BDNF, GDF-5, BMP-7 and IL-6.

In another aspect of the invention, cells **22** in cell sheet layer **20,** and their progeny, can be engineered to continually produce a desired gene product. In another aspect, the cells may only transiently produce a product. Methods of transforming or transfecting cells with an exogenous polynucleotide, such as a DNA molecule, are well known in the art and include techniques such as calcium phosphate- or DEAE-dextran-mediated transfection, protoplast fusion, electroporation liposome-mediated transfection, direct microinjection and adenovirus infection. The most widely used method is transfection mediated by either calcium phosphate or DEAR-dextran. Methods of permanent and transient transfections are known and will he described further herein. One particular advantage of utilizing a tissue sheet comprising genetically engineered cells is that a large amount of cells may be utilized compared to simply seeding a relatively few number of genetically engineered cells. In another preferred embodiment, a cell preparation graft would include a plurality of modified cell types, each producing a different therapeutic substance at a desired therapeutic level. This embodiment is useful as a screen for drug development.

In another aspect of the invention, cell sheet layer **20** comprised of cells **22** can be reinforced with exogenously added extracellular matrix proteins, e.g. collagens, laminins, fibronectin, vitronectin, tenascin, integrins, glycosaminoglycans (hyaluronic acid, chondroitin sulfate, dermatan sulfate, heparan sulfate, heparin, keratan sulfate and the like), elastin and fibrin. Cell sheet layer **20** can incorporate exogenously added growth factors and/or cytokines, such as vascular endothelial cell growth factors, platelet derived growth factors, epidermal growth factors, fibroblast growth factors, hepatocyte growth factors, insulin-like growth factors and transforming growth factors.

Selected cells **22** may be cultured on a surface of glass, ceramic or a surface-treated synthetic polymer. For example, polystyrene that has been subjected to a surface treatment, like gamma-ray irradiation or silicon coating, may be used as a surface for cell culture. Cells **22** grown to over 85% confluence form cell sheet layer **20** on cell growth support device **14.** Cell sheet layer **20** may be separated from cell growth support device **14** using proteolysis enzymes, such as trypsin or dispase. Non-enzymatic cell dissociation could also be used. A non-limiting example includes a mixture of chelators sold under the tradename CELLSTRIPPER (Mediatech, Inc., Herndon, VA), a non-enzymatic cell dissociation solution designed to gently dislodge adherent cells in culture while reducing the risk of damage associated with enzymatic treatments.

Alternatively, the surface of cell growth support device **14,** from which cultured cells **22** are collected, may be a bed made of a material from which cells detach without a proteolysis enzyme or chemical material. The bed material may comprise a support and a coating thereon, wherein the coating is formed from a polymer or copolymer which has a critical solution temperature to water within the range of 0°C to 80°C.

The critical solution temperature is defined as follows. When a certain material is mixed with water, the mixture is divided into two layers at a particular temperature because of its poor solubility, but eventually the material is completely dissolved with water to turn it to a uniform solution if it is either heated or cooled beyond a certain temperature. The certain temperature is defined as "critical solution temperature". If the uniform solution is formed when heated, the critical solution temperature is called "upper critical solution temperature". If the uniform solution is formed when cooled, it is called the "lower critical solution temperature".

The polymer or copolymer should have either an upper or lower critical solution temperature within the range of 0° to 80°C, preferably 20° to 50°C. If it is higher than 80°C, cultured or grown cells may die. If it is lower than 0°C, the growth rate of the cells may be very much lowered or the cells may die.

The polymer or copolymer may be prepared by polymerizing or copolymerizing some hydrophilic monomers. Non-limiting examples of the monomers are represented by an acrylamide, such as methacrylamide; an N-substituted acrylamide or methacrylamide, such as N-ethyl acrylamide, N-n-propyl acrylamide, N-n-propyl methacrylamide, N-isopropyl acrylamide, N-isopropyl methacrylamide, N-cyclopropyl acrylamide, N-cyclopropyl methacrylamide, N-ethoxyethyl acrylamide, N-ethoxyethyl methacrylamide, N-tetrahydrofurfuryl acrylamide, N- tetrahydrofurfuryl methacrylamide; an N,N-di-substituted acrylamide or methacrylamide, such as N,N-dimethyl acrylamide, N,N-dimethyl methacrylamide, N.N-ethylmethyl acrylamide, N,N-diethyl acrylamide; 1-(1-oxo-2-propenyl)-pyrrolidine, 1-(1-oxo- 2-propenyl)-piperidine, 4-(1-oxo-2-propenyl)-morpholine, 1-(1-oxo-2-methyl-2-propenyl)-pyrrolidine, 1-(1-oxo-2-methyl-2-propenyl)-piperidine, 4-(1-oxo-2-methyl-2-propenyl)-morpholine etc.; a vinyl ether, such as methyl vinyl ether; and the like.

Additional monomers include acrylamide derivatives of the following general formula (I): 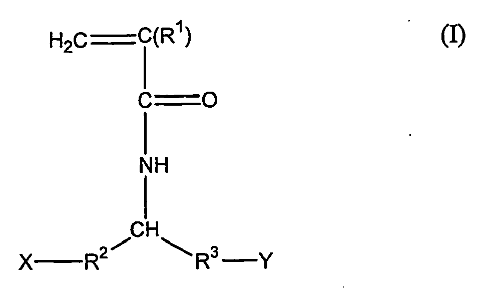 wherein R¹ represents a hydrogen atom, a straight-chain or branched alkyl group containing 1 to 6 carbon atoms or a C3-6 cycloalkyl group, R² and R³ each independently represent an alkylene group containing 1 to 6 carbon atoms or R² and R³ may be combined to form a ring, X represents a hydrogen atom, an amino group, a hydroxyl group, a halogen atom, a carboxyl group or a -COOR⁴ group wherein R⁴ represents a C1-6 straight-chain or branched alkyl, C3-6 cycloalkyl, phenyl, substituted phenyl, benzyl or substituted benzyl group, and Y represents an amino group, a hydroxyl group, a halogen atom, a carboxyl group or a -COOR⁴ group wherein R⁴ represents a C1-6 straight- chain or branched alkyl, C3-6 cycloalkyl, phenyl, substituted phenyl, benzyl or substituted benzyl group.

The polymer may also consist of identical or different repeating units of the following general formula (II): 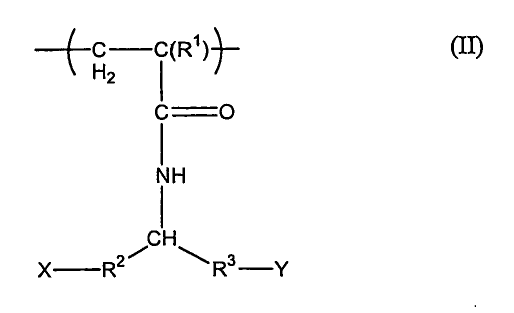 wherein R¹ represents a hydrogen atom, a straight-chain or branched alkyl group containing 1 to 6 carbon atoms or a C3-6 cycloalkyl group, R² and R³ each independently represent a alkylene group containing 1 to 6 carbon atoms or R² and R³ may be combined to form a ring, X represents a hydrogen atom, an amino group, a hydroxyl group, a halogen atom, a carboxyl group or a - COOR⁴ group wherein R⁴ represents a C1-6 straight-chain or branched alkyl, C3-6 cycloalkyl, phenyl, substituted phenyl, benzyl or substituted benzyl group, and Y represents an amino group, a hydroxyl group, a halogen atom, a carboxyl group or a -COOR⁴ group wherein R⁴ represents a C1-6 straight-chain or branched alkyl, C3-6 cycloalkyl, phenyl, substituted phenyl, benzyl or substituted benzyl group.

The copolymer may also consist of different or identical repeating units of the above general formula (II) and different or identical repeating units of general formula (III): 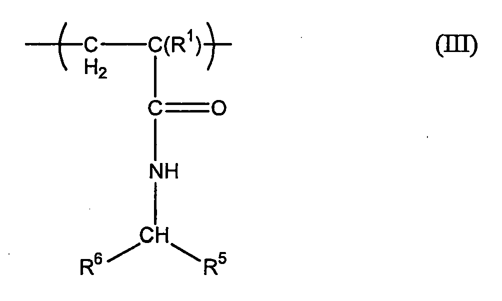 wherein R¹ represents a hydrogen atom, a straight-chain or branched alkyl group containing 1 to 6 carbon atoms or a C3-6 cycloalkyl group, R⁵ represents a straight-chain or branched alkyl group containing 1 to 6 carbon atoms or a C3-6 cycloalkyl group, and R⁶ represents a straight-chain or branched alkyl group containing 1 to 6 carbon atoms or a C3-6 cycloalkyl group, or R⁵ and R⁶ may be combined to form a 3-, 4-, 5 or 6-membered ring in which the -CH- group to which they are attached is one member.

A copolymer of the above listed monomers or other monomers, a graft polymer or copolymer or a mixture of the polymers can also be employed in order to adjust the critical solution temperature, to enhance an interaction between the support and the coating thereon or to control the balance between the hydrophilic and hydrophobic properties of the bed material. The polymer or copolymer of the present invention may be crosslinked unless the inherent properties of the polymer would be deleteriously affected thereby.

The support can be prepared from any material for example polymers such as polystyrene, poly(methyl methacrylate), polyvinylidene difluoride (PVDF), polypropylene, polyethylene, vinyl polymers; ceramics, metals, glass and modified glass. The shape of cell growth support device **14** is not limited, but typically a Petri dish, a plate, a fiber, particles or any type of container (e.g. a flask) can be used for the cell culture.

A polymer or copolymer can be bound on the support by a chemical method or by a physical method. In the chemical method, an electron beam, gamma-ray irradiation, ultraviolet irradiation, corona treatment or plasma treatment can he used. In case where the support and the coating have groups reactive with each other, an organic reaction (e.g. a radical, anionic or cationic reaction) can also be used. In the physical method, the polymer per se or a combination of the polymer and a matrix compatible with the support is coated on the support, thus binding by physical absorption power. Examples of the matrix are graft, or a block copolymer of the polymer to be coated, with the monomer forming the support or other monomers compatible with the support.

In order to collect or detach the grown or cultured cell sheet layer **20,** the bed material is either heated or cooled to exceed the upper or lower critical solution temperature, thus detaching cell sheet layer **20,** and rinsed with an isotonic solution to collect cell sheet layer **20.**

In another embodiment, cell sheet layer **20** is produced by culturing cell **22** on a non-adherent surface at sufficient densities. This provides a cell sheet layer **20** that has only a few structural defects as they are recovered with intracellular desmosome structures and the cell-to-cell connectivity and orientation being kept intact.

In this embodiment, cell growth support, device **14** can be inherently non-adherent or can be rendered non-adherent by surface coatings well known to those skilled in the art. Commercially available cell growth support devices include, for example, the range of Corning® Ultra Low Attachment surface cell culturing products (Corning Inc., Corning NY). These products have a hydrogel layer that is hydrophilic and neutrally charged covalently bound to polystyrene surfaces. Since proteins and other biomolecules passively adsorb to polystyrene surfaces through either hydrophobic or ionic interactions, this hydrogel surface naturally inhibits non-specific immobilization via these forces, thus inhibiting subsequent cell attachment. Other biocompatible non-adherent materials include ePTFE, polystyrene, stainless steel and some cross-linked cellulose derivatives. Examples thereof include cross-linked hydroxyalkyl celluloses e.g. hydroxyethyl cellulose, hydroxypropyl cellulose, methyl, ethyl and methyl ethyl celluloses. Cross-linked carboxyalkyl celluloses also included are carboxymethyl cellulose cross-linked with ethylene glycol diglycidyl ether (EGDGE) or 1,4 butanediol diglycidyl ether. Other materials include polyvinyl alcohol, poly(2-hydroxyethyl methacrylate) (Cellform® (MP Biomedicals, Irvine, CA)), agarose and crosslinked agarose.

To produce cell sheet layer **20**, a cell population **22** (homogenous or heterogeneous) is cultured on a non-adherent substrate in the presence of commonly available culture media components, to promote extracellular matrix protein production. After an extended culture period, enough extracellular matrix protein is produced to make a coherent cell sheet.

Cells can be seeded on cell growth support device **14** at different densities sufficient to permit the formation of cell sheet layer **20.** This will vary for different cell types and will need to be optimized. In the case of chondrocytes, the cell densities can range from 1,000 cells/cm² to 100,000 cells/cm². Appropriate culture medium (for example, D-MEM medium, MEM medium, HamF12 medium, HamF10 medium) is added to support device **14.** Cells **22** of the required density are then added to support device **14** so that cells **22** settle to the bottom of the dish, Alternatively cells **22** of the required number can be suspended in the culture medium and added to support device **14**. In this case, cells will not attach to the bottom of the dish and cell-to-cell adhesion creates cell sheet layer **20.** Cultures are maintained for a few days to a few weeks before cell sheet layer **20** can be recovered from support device **14**. During culture, the culture medium may be exchanged, if needed. Usually, the culture medium is exchanged every 0.6 to 2 days of the culture. The addition of agents that promote cell growth, viability and/or cell-to-cell adhesion can be used during the culture process. For example, addition of agents such as ascorbic acid, retinoic acid and copper can be used to increase the production of extracellular matrix proteins, thereby generating a more robust sheet layer. Growth factors capable of stimulation of extracellular matrix protein production or a combination of growth factors, microelements, vitamins and such could be used.

Cell sheet layer **20** produced on the cell growth support device **14** can be recovered from support device **14** by gently peeling cell sheet layer **20** using a pair of forceps. Alternatively, cell sheet layer **20** is brought into close contact with a polymer membrane such that cell sheet layer **20** adheres to the polymer sheet. The coupled cell sheet/polymer backing can then be removed with a pair of tweezers. Peeling of cell sheet layer **20** can bo performed not only in the culture solution used to culture cells **22,** but also in other isotonic solutions. A suitable solution can be chose in accordance with a specific object. Examples of the polymer membrane that can be used to achieve close contact with cell sheet layer **20** include polyvinylidene difluoride (PVDF), polypropylene, polyethylene, cellulose and its derivatives, as well as chitin, chitosan, collagen, polyurethane and other such films or meshes made with known bioresorbable natural and synthetic polymers. The backing layer may be continuous or apertured (formed into a net). The backing layer may be flat or contoured. The contours may be produced, for example, by embossing. Suitably contoured films may also have apertures.

In another embodiment, cell sheet layer 20 can be comprised of reinforced cell sheets. Reinforced cell sheets can be formed by placing biodegradable or non-biodegradable reinforcing members at the bottom of support device **14** prior to seeding support device **14** with cells **22.** Reinforcing members include, but are not limited to, textile structures such as weaves, knits, braids, perforated films, meshes and non-wovens. Cell sheet layers **20** that result will have incorporated the reinforcing member, providing additional strength to cell sheet layer **20,** which can be manipulated without the requirement for a backing layer. A preferred reinforcing member is a knitted or non-woven mesh comprised of poly(glycolic acid-co-lactic acid) copolymer (PGA/PLA) fibers sold under the tradename VICRYL (Ethicon, Inc., Somerville, NJ). The mesh can be placed at the bottom of the Corning® Ultra low attachment dish. Cells can then be seeded on to the dishes such that they will form cell-cell interactions but also bind to the mesh when they interact with the mesh. This will give rise to reinforced cell sheets with better strength and handling characteristics. Such reinforced cell sheets will not require additional backing polymer or scaffold to aid in removal.

Cell sheet layer **20** can also be made with a ring of the mesh such that the center of the cell sheet has no mesh and only the surrounding is reinforced with the mesh.

Cell sheet layer **20** is disposed on a scaffold layer **30** in the method of the invention. Scaffold layer **30** is a three-dimensional scaffold, or framework. Scaffold layer **30** may be configured into various shapes such as generally flat, generally cylindrical or tubular, or can be completely free-form as may be required or desired for the corrective structure under consideration. When grown in this three-dimensional system, the proliferating cells from cell sheet layer **20** mature and segregate properly to form components of adult tissues analogous to counterparts found naturally *in vivo.*

For example, but not by way of limitation, scaffold layer 30 may be designed such that the scaffold structure: (1) supports the cell sheet without subsequent degradation; (2) supports cell sheet from the time of seeding until the tissue transplant is remodeled by the host tissue; (3) allows the seeded cell sheet to attach, proliferate, and develop into a tissue structure having sufficient mechanical integrity to support itself *in vitro*, at which point, the matrix is degraded.

Examples of scaffolds that may be used for aspects of the invention include textile structures such as weaves, knits, braids, meshes, non-wovens and warped knits; porous foams, semi-porous foams, and perforated films or sheets and composite structures being a combination of the above structures. Non-wovcn mats may, for example, be formed using fibers comprised of natural or synthetic polymers.

Scaffold layer 30 of the present invention is preferably formed from a biocompatible polymer. A variety of biocompatible polymers can be used to form the mats and foams according to the present invention. The biocompatible polymers can be synthetic polymers, natural polymers or combinations thereof. As used herein, the term "synthetic polymer" refers to polymers that are not found in nature, even if the polymers are made from naturally occurring biomaterials. The term "natural polymer" refers to polymers that are naturally occurring.

Biocompatible polymers for use in the present invention may be biodegradable or non-biodegradable. Biodegradable polymers readily break down into small segments when exposed to moist body tissue. The segments then are either absorbed by the body or passed by the body. More particularly, the biodegraded segments do not elicit permanent chronic foreign body reaction, because they are absorbed by the body or passed from the body, such that no permanent trace or residue of the segment is retained by the body.

In embodiments where the scaffold includes at least one synthetic polymer, suitable biocompatible synthetic polymers can include polymers selected from the group consisting of aliphatic polyesters, poly(amino acids), copoly(ether-esters), polyalkylene oxalates, polyamides, tyrosine-derived polycarbonates, poly(iminocarbonates), polyorthoesters, polyoxaesters, polyamidoesters, polyoxaesters containing amine groups, poly(anhydrides), polyphosphazenes, poly(propylene fumarate), polyurethane, poly(ester urethane), poly(ether urethane) and blends and copolymers thereof. Suitable synthetic polymers for use in the method of the present invention can also include biosynthetic polymers based on sequences found in collagen, laminin, glycosaminoglycans, elastin, thrombin, fibronectin, starches, poly(amino acid), gelatin, alginate, pectin, fibrin, oxidized cellulose, chitin, chitosan, tropoelastin, hyaluronic acid, silk, ribonucleic acids, deoxyribonucleic acids, polypeptides, proteins, polysaccharides, polynucleotides and combinations thereof.

For the purpose of this invention, "aliphatic polyesters" include, but are not limited to, homopolymers and copolymers of monomers including lactide (which includes lactic acid, D-, L- and meso lactide); glycolide (including glycolic acid); epsilon-caprolactone; p-dioxanone (1,4-dioxan-2-one); trimethylene carbonate (1,3-dioxan-2-one); alkyl derivatives of trimethylene carbonate; and blends thereof. Aliphatic polyesters used in the present invention can be homopolymers or copolymers (random, block, segmented, tapered blocks, graft, triblock, etc.) having a linear, branched or star structure.

In embodiments where the scaffold includes at least one natural polymer, suitable examples of natural polymers include, but are not limited to, fibrin-based materials, collagen-based materials, hyaluronic acid-based materials, glycoprotein-based materials, cellulose-based materials, silks and combinations thereof. By way of non-limiting example, the biocompatible scaffold can include a collagen-based small intestine submucosa, periosteal membrane, synovial or amniotic membrane.

One skilled in the art will appreciate that the selection of a suitable material for forming the biocompatible scaffold layer **30** depends on several factors. These factors include *in vivo* mechanical performance; cell response to the material in terms of cell attachment, proliferation, migration and differentiation; biocompatibility; and optionally, biodegradation kinetics. Other relevant factors include the chemical composition, spatial distribution of the constituents, the molecular weight of the polymer and the degree of crystallinity.

Foams composed of, for example, poly(epsilon-caprolactone-co-glycolic acid) copolymer, formed by processes such as freeze-drying or lyophilization, as discussed in U.S. Patent No. 6,355,699, can serve as scaffold **30.** In another preferred embodiment, cell sheets 20 of the invention are seeded onto foam scaffolds **30** that may be composite structures of fiber reinforced foams.

Textiles, including weaves, non-wovens, knits, warped knits (i.e., lace-like structures) and braids, can be used as scaffold **30.** For example, the reinforcing component has a mesh-like structure. The fibers used to make the textile structures can be monofilaments, yarns, threads, braids or bundles of fibers. These fibers can be made of any biocompatible material including synthetic biodegradable materials such as polylactic acid (PLA), polyglycolic acid (PGA), polycaprolactone (PCL), polydioxanone (PDO), polytrimethylene carbonate (PTMC), polyvinyl alcohol (PVA) and copolymers or blends thereof. The fibers can be formed of a polylactic acid and polyglycolic acid copolymer at a 95:5 mole ratio. The fibers can alternatively be made of a bioabsorbable glass. Bioglass, a silicate containing calcium phosphate glass, or calcium phosphate glass with varying amounts of iron particles added to control resorption time are examples of materials that could be spun into glass fibers and used for the textile structures. Also, filaments that form the fibers may be co-extruded to produce a filament with a sheath/core construction. Such filaments are comprised of a sheath of biodegradable polymer that surrounds one or more cores comprised of another biodegradable polymer. Filaments with a fast-absorbing sheath surrounding a slower-absorbing core may be desirable in instances where extended support is necessary for tissue ingrowth. Bioresorbable metals could be used as well.

Scaffold **30** may be a felt, which can be composed of a multifilament yarn made from a bioabsorbable material, *e.g.* PGA, PLA, PCL copolymers or blends, or hyaluronic acid. One yarn of interest is made of poly(glycolic acid-co-lactic acid) copolymer or PLGA, sold under the tradename VICRYL (Ethicon, Inc., Somerville, NJ) and is used to form a mat. The yarn is made into a felt using standard textile processing techniques consisting of crimping, cutting, carding and needling.

Scaffold **30** may be formed from thin, perforation-containing polymer sheets with perforations to allow tissue ingrowth. Such sheets could be made of the same polymers and copolymers as described above for scaffold materials.

One of ordinary skill in the art will appreciate that one or more layers of the scaffold **30** may be used in tissue engineering device **10.** In addition, scaffold layers **30** (e.g. meshes) of the same structure and chemistry or different structures and chemistries can be overlaid on top of one another to fabricate tissue engineering devices **10** with superior mechanical strength. The scaffold **30** might provide additional benefit since it might incorporate a genetic material, cytokines and growth factors to promote survival, proliferation and differentiation of cells **22**. Various means of incorporation of these beneficial factors into the scaffold **30** are known to those skilled in the art including, but not limited to, coating,

Also, cells sheet layers **20** composed of different selected cells **22** can be overlaid on top of one another to fabricate tissue engineering devices **10** with superior cellular performance. Multilayered cell sheets can be made in several ways. For example, a first cell sheet layer of a first cell type is laid down on a non-adherent surface; then, a second cell sheet layer of a second cell type is laid down on the first cell sheet layer and allowed to adhere to the first cell sheet layer. The kinds of cells of the cell sheet layers to be laminated may be the same or different. For example, a cellular sheet or multi-layered sheet could be made of bladder-derived urothelial cells and positioned on a luminar surface of the scaffold and smooth muscle cell sheet positioned on an external surface of the scaffold. In addition, keratinocytes may be overlaid with a fibroblast sheet and/or angioendothelial cell sheet that have been prepared by similar operations. This technique is extremely effective in realizing a product even nearer to an in vivo skin tissue. Sprinkling the sheet with endothelial cells, for example, helps to grow vascular networks (vasovasorum).

The number of cell sheet layers to be laminated is not limited. Generally the number cell sheet layers to be laminated is from 1 to 10, preferably from 1 to 5, more preferably from 1 to 3.

Scaffold **30** may be molded into a useful shape, such as a specific structure in the body to be repaired, replaced or augmented.

The tissue engineering device **10** contemplated for use herein can be implanted in combination with any one of growth factors, drugs or other components, such as bioactive agents that promote healing or ingrowth of tissue, or stimulate vascularization or innervation or immunomodulatory agent thereof or otherwise enhance or improve the therapeutic outcome or the practice of the invention, in addition to cell sheets **20** of the invention.

In some embodiment it is useful to re-create in culture the cellular microenvironment found *in vivo*, such that the extent to which the cells arc grown prior to implantation *in vivo* or use *in vitro* may vary. Cell sheets **20** may be disposed onto scaffold **30** before or after forming the shape desired for implantation, *e*.*g*. ropes, tubes, filaments and the like. Following disposition of cell sheet **20** onto scaffold **30**, scaffold **30** is preferably incubated in an appropriate growth medium. During the incubation period, cells **22** in cell sheet **20** will grow and envelop scaffold 30 and may, for example, bridge, or partially bridge, any interstitial spaces therein, It is preferable, but not required, to grow the cells to an appropriate degree that reflects the *in vivo* cell density of the tissue being repaired or regenerated.

In another aspect, cell sheet layer **20** of the invention is substantially decellularized to provide extracellular matrix materials provided by the population of cells. In some cases, it may be advantageous to decellularize or denature all or part of the tissue engineered device. A decellularized sheet may have a reduced level of immunogenicity and may provide a better matrix. Decellularizing or denaturing tissue engineering device **10** may also enhance the mechanical characteristics of the device. Cell sheet layer **20** may be decellularized, denatured or chemically modified using a variety of techniques. In the simplest embodiment, tissue engineering device **10** can be air-dried or lyophilized to kill the cells. Thermal shock, acoustic treatment, changes in pH, osmotic shock, mechanical disruption or addition of toxins can also induce cell death or apoptosis. Similarly, tissue engineering device **10** can be cross-linked or fixed using agents such as paraformaldehyde. Other treatments to decellularize or denature the tissue are possible using radiation, detergents (SDS or triton x100), enzymes (RNAasc, DNAase) or solvents (alcohol, acetone, or chloroform). Treatment with hypotonic and/or hypertonic solutions, which have nonphysiological ionic strengths, can promote the decellularization process. These various decellularization solutions generally are suitable as treatment solutions. Proteases also can be used effectively to decellularize tissue. These techniques are only some of the examples of techniques to decellularize, denature or chemically modify all or part of the tissue and are not meant to limit the scope of the invention.

The decallularization can be performed in stages with some or all of the stages involving differential treatments. For example, a potent mixture of proteases, nucleases and phospholipases could be used in high concentrations to decellularize the tissue. The decellularized extracellular matrix may then have applied another tissue sheet or another decellularized sheet. For example, one can roll a living layer on top of a decellularized layer.

Tissue engineering device **10** produced using this novel superior method can be an integral part of the new field of tissue engineering. Device **10** may be used to bioengineer tissue substitutes for other target tissues and organs in the body, including but not limited to, spinal cord, nerves, bone, cartilage, ligaments, tendons, heart valves, esophagus, cervix, bladder, lung tissue, kidney, skin, fascia, and other tissues. Create skin, blood vessels, myocardial patches, vascular patches, heart valves, and more complex organs. The tissue engineering device **10** could function on ist own or could be a component of another system, For example based on a cell type it could provide an element of an extracorporeal devices simulating a specific organ function. For example, an extracorporeal kidney filtration unit. This tissue engineering device could function as a reservoir of functional beneficial proteins i.e. EPO.

Tissue engineering device **10** could be supplied crypreserved or freeze-dried by using excipients known in the art such as trehalose, glycerol, sorbitol.

Tissue engineering device **10** allows for the first time the production of mechanically sound living tissues and organs, that can be made exclusively from cell sheets. Furthermore, sheet-based tissue engineering opens the door to the production or tissues and organs made from patients own cells, avoiding all rejection complications.

The three-dimensional tissue structure can be also be used for, for example, in vitro permeability tests of drugs, and can also be utilized as a substitute model for an animal experiment or as an organ for transplantation.

### Example 1:

Cells were subjected to cell sheet preparation and were combined with non-woven, degradable scaffolds as a first step towards generation of tissue engineering devicces. Osteoblasts/chondrocytes for musculo-skeletal applications as well as urothelial cells/bladder smooth muscle cells for uro-genital applications were tested. Cells were cultured prior to scaffold deposition.

Porcine urothelial cells were isolated as described in Rahman, Z et al., "Isolation and primary culture urothelial cells from normal human bladder," Urol. Res. 15 (1987) (6), pp. 315-320. Cells were cultured in Keratinocyte Serum Free Medium (Invitrogen, Carlsbad, CA) in tissue culture plastic flasks. The cultures were incubated at 37° C with 5 % CO₂. Cells used for experiments were between passages 1 and 2.

Human bladder smooth muscle cells were purchased from Cambrex (CC-2533; Cambrex, Walkersville, MD). Cells were cultured in muscle cell growth medium, SmGM-2 (CC-3182; Cambrex, Walkersville, MD) in tissue culture plastic flasks. The cultures were incubated at 37° C with 5 % CO₂. Cells used for experiments were between passages 3 and 5.

Human osteoblast cells were purchased from Cambrex (CC-2533, Cambrex, Walkersville, MD). Cells were cultured in smooth osteoblast growth medium, OGM (CC-3207; Cambrex, Walkersville, MD) in tissue culture plastic flasks. The cultures were incubated at 37° C with 5 % CO₂. Cells used for experiments were between passages 3 and 4.

Bovine cartilage tissue was harvested from femoral condyles of adult bovine animals. Primary bovine chondrocytes were isolated from cartilage tissue by O/N collagenase digestion at 4°C. Cells were cultured in chondrocyte growth medium, CGM (CC-3216; Cambrex, Walkersville, MD) in tissue culture plastic flasks. The cultures were incubated at 37° C with 5 % CO₂. Cells used for experiments were between passages 3 and 4.

The cell types described above were also cultured according to the invention in sheets on thermoresponsive dishes. Cells were seeded on **35** millimeter p(NIPAAM)-coated dishes supplied by CellSeed, Inc. (Tokyo, Japan). CellSeed, Inc. manufactures several types of dishes from A to G varying in the degree of cellular attachment or detachment. Dishes range from good cellular attachment to good cellular detachment to strong cellular detachment. Cells were grown to confluency as shown in Table 1 below.

**Table 1: Time to confluence and thermoresponsive dish type are listed for the appropriate cell type.**

| Cell type | Dish type | Time to confluence |
|---|---|---|
| Chondrocytes | C at 10,000 cells/cm² | 6 days |
| Osteoblasts | D at 10,000 cells/cm² | 7 days |
| Urothelial cells | D at 10,000 cells/cm² | 8 days |
| SMCs | B at 10,000 cells/cm² | 5 days |

The scaffold material was the same for all of the treatment groups and consists of a PGA non-woven mesh at 55 grams/cubic centimeter and thickness of 2 millimeters. Fiber size is 12-15 microns. Scaffolds were coated with a 5% solution of 50/50 mol percent PLGA (Sigma, St. Louis, MO, Mw 40,000-75,000) in methylene chloride (Sigma Aldrich, St. Louis, MO), which made them stiff. Scaffolds were cut in the form of 35 millimeter diameter circles and were sterilized using ethylene oxide sterilization. Next, the cell sheets were co-cultured with the scaffold. In the case of single layer cell sheets, the cell sheet for the first cell type was embedded by adding 500 microliters of fibrinogen (80 milligrams/milliliter in phosphate buffered saline (PBS) (Sigma, St. Louis, MO) and gelatinized with 400 microliters of bovine thrombin solution (Jones Pharma, Inc., Middleton, WI) while still in the thermoresponsive dish. The embedded cell sheet was then layered on top of the scaffold in a 100 millimeter tissue culture polystyrene dish. 30 milliliters of growth medium were added to the dish and the dish was placed in an incubator for two days. The appropriate medium was used for the given cell type as shown in Table 2 below. The medium was changed twice daily. After incubation, the device was turned over and the process was repeated on the other side of the scaffold with the second cell type. 30 milliliters of growth medium were added to the dish and the dish was placed in an incubator for four days. Therefore, the total incubation time for the scaffold devices was 6 days.

**Table 2: The appropriate cell growth medium for cell types during scaffold deposition.**

| Cell Type | Growth Medium |
|---|---|
| Chondrocytes alone | Osteoblasts growth medium (cat#: CC-3207, Cambrex, Walkersville, MD) supplemented with non-essential amino acids (NEAA, cat#: 11140-050, Invitrogen, Carlsbad, CA) at a concentration of 0.1 millimolar and HEPES buffer (cat#: 15630-080, Invitrogen, Carlsbad, CA) at a concentration of 10 millimolar |
| Chondrocytes and osteoblasts | Osteoblasts growth medium (cat#: CC-3207, Cambrex, Walkersville, MD) |
| Bladder smooth muscle cells alone | Dulbeco Modified Eagle's Medium (DMEM), 10% fetal bovine serum, penicillin/streptomycin (Invitrogen, Carlsbad, CA) |
| Bladder smooth muscle cells with urothelial cells | 50:50 mixture ofKeratinocyte Serum Free Medium and DMEM, 10% FBS, penicillin/streptomycin (Invitrogen, Carlsbad, CA) |

In the case of multi-layer cell sheets, the first cell sheet was embedded and gelatinized as described above for single sheets. The cell sheet was transferred on top of a second cell sheet in a thermoresponsive dish and the two sheets were lifted out of the dish and transferred on top of another sheet. The cell sheets were layered in this manner until the desired number of sheets was obtained. Once the desired number of sheets was obtained the multi-layer sheet was incubated for approximately 45 minutes then transferred onto a scaffold in a 100 millimeter tissue culture polystyrene dish, embedded with 500 microliters of fibrinogen (80 milligrams/milliliter in PBS) and gelatinized with 400 microliters of thrombin solution. 30 milliliters of growth medium were added to the dish and the dish was placed in an incubator for two days. The appropriate medium was used for the given cell type as shown in Table 2. The medium was changed twice daily. After incubation, the device was turned over and the process was repeated on the other side of the scaffold with the second cell type. 30 milliliters of growth medium were added to the dish and the dish was placed in an incubator for four days. The total incubation time for the scaffold devices was 6 days.

For comparison, cell suspensions were seeded on the scaffold such that the cell number was matched to corresponding cell sheets as shown in Table 3. The cells were resuspended in 1 milliliter of medium. The 1 milliliter of cell suspension was placed in the middle of the scaffold and incubated for 45 minutes to allow cell attachment to the scaffolds. The appropriate medium was used for the given cell type as shown in Table 2. The medium was changed twice daily. After incubation, the device was turned over and the process was repeated on the other side of the scaffold with the second cell type. Then, 30 milliliters of medium was added to the dish and placed in an incubator for four days. The total incubation time for the scaffold devices was 6 days.

**Table 3: Seeding density for cell sheets as well as viability of cells seeded onto the scaffolds.**

| Cell type | Number of cells seeded | Viability |
|---|---|---|
| Chondrocytes | 1.53x10⁶ (two sheets) | 85% |
| Osteoblasts | 6.45x10⁵ (two sheets) | 80% |
| Urothelial cells | 5.03x10⁵ (one sheet) 1.51x10⁶ (3 sheets) | 84% |
| SMCs | 4.35x10⁵ (one sheet) 1.30x10⁶ (3 sheets) | 92% |

The following cell/scaffold devices were generated using the methods described above. Devices 1 to 6 were made according to the method of the invention. Device 7 was made according to a method of the prior art.
1. One Urothelial cell sheet + Scaffold + one Bovine Smooth Muscle (BSM) cell sheet
2. One cell sheet equivalent of Urothelial cell suspension + Scaffold + one cell sheet equivalent of BSM cell suspension
3. Two Osteoblasts cell sheets + Scaffold + two Chondrocytes cell sheets
4. Two cell sheet equivalents of Osteoblasts cell suspension + Scaffold + two cell sheet equivalents of Chondrocytes cell suspension
5. Three Urothelial cell sheets + Scaffold + three Bovine Smooth Muscle (BSM) cell sheets
6. Three cell sheet equivalents of Urothelial cells suspension + Scaffold + three cell sheet equivalents of BSM cell suspension
7. Scaffold alone

Three days after the last cell deposition, the devices were cut into approximately 0.5 cm² pieces using a scalpel. The pieces were embedded in fibrin glue by adding 30 microliters of fibrinogen (80 milligrams/milliliter in PBS) and gelatinized with 5 microliters of bovine thrombin solution (Jones Pharma, Inc., Middleton, WI). The embedded and gelatinized device pieces were stored at 37°C in the culture medium overnight. The device pieces were removed from the culture medium and washed with PBS and characterized by live/dead staining and histology.

Live/dead staining allows visualization of cells remaining in the scaffolds after all manipulations as well as to examine the degree of cell survival within the scaffolds. Cells were exposed to 1 micromolar Calcein AM (Molecular Probes, Eugene OR) in PBS (live staining) and 1 micromolar Ethidium (Molecular Probes, Eugene OR) in PBS (dead staining) concurrently for 1 hour at room temperature. Following staining, fluorescence was visualized using the appropriate fluorescence filter on an Olympus inverted epi-fluorescent microscope (Olympus, Melville, NY). Representative images were captured using a digital color videocamera and ImagePro software (Media Cybernetics, Carlsbad, CA). The majority of stained cells were viable with only a few dead cells. Furthermore, the live cells were dispersed throughout the scaffold without apparent difference between the two seeding methods.

Histology samples were fixed in 10% formalin and were sent to Paragon Bioservices (Baltimore, MD) for paraffin embedding, sectioning, and staining with Hematoxylin /Eosin (H/E) and Safranin O (SO). H/E staining also confirmed that cells were present throughout the scaffolds regardless of seeding method. Furthermore, for scaffolds seeded with one cell sheet versus three cell sheets (urothelial cells/BMS cells) there was no apparent difference in cell distribution. Safranin O staining revealed similar results. No Safranin O positive areas were detected in any of the examined scaffolds. This suggests that *in vitro* culture of cell/scaffold devices was not sufficient for generation of detectable levels of proteoglycans, regardless of seeding method.

Cells were subj ected to cell sheet preparation and were combined with non-woven, degradable scaffolds as a first step towards generation of tissue engineering devices. Two cell systems were tested: osteoblasts/chondrocytes for potential regenerative medicine musculo-skeletal systems as well as urothelial cells/bladder smooth muscle cells for uro-genital applications. The seeding of cells on scaffolds using cell sheets was compared against the conventional method of seeding scaffolds with cell suspension. Seeding of a biodegradable, biocompatible scaffold with cell sheets provides equivalent cell viability and cell distribution to seeding by cell suspension. Therefore, seeding scaffolds using the cell sheet method is a good alternative to the conventional method of cell suspension seeding with the benefit of one-step application and more controlled device handling.

### Example 2:

An *in vivo* implantation pilot study was performed using the tissue engineering devices prepared in Example 1 in a SCID mouse subcutaneous model. Twenty Fox Chase SCID CB17SC/male mice were ordered from Taconic Inc. (Germantown, NJ). The average age of the animals at the time of the study was 5 weeks. Animals were selected without any apparent systematic bias. Duration of the study was 4 weeks.

The devices were implanted subcutaneously by making two skin incisions, each approximately 5 mm in length, on the dorsum of the mice. The incisions were placed transversely over the lumbar area about 5 mm cranial to the palpated ilcac crest, with one on either side of the midline. Devices with the same treatment were placed in the two sites. The skin was separated from the underlying connective tissue to make a small pocket, and the implant was placed about 10 mm cranial to the incision. The skin incisions were closed with Reflex 7 metal wound clips (CellPoint Scientific, Inc., Gaithersburg, MD).

All mice survived the surgery and the duration of the study. Mice were euthanized by CO₂ inhalation at 4 weeks post-implantation. Gross observations of the implanted sites were recorded. The subcutaneous implantation sites with their overlying skin were excised. With the exception of scaffolds seeded with osteoblasts/chondrocytes, subcutaneous implantation sites with their overlying skin were bisected. Half of the tissue was preserved in 10% buffered formalin fixative for paraffin embedding and histological staining (H&E). Another half was frozen for immunohistochemical staining (pancytokeratin, keratin 7, anti-alpha smooth muscle actin). The samples will be evaluated histologically for retention of cell phenotypes, cellular infiltration into the scaffold and vascularization. Scaffolds seeded with osteoblasts/chondrocytes were preserved in 10% buffered formalin fixative for paraffin embedding and histological staining only (H&E and Safranin O).

The gross observations were noted as follows: upon sacrifice at 4 weeks post-implantation the implants appeared firm to the touch, which indicated tissue ingrowth. The implants maintained their shape and in some cases were partially resorbed. Implants also appeared pinkish in color, which indicated vascularization.

Although this invention has been shown and described with respect to detailed embodiments thereof, it will be understood by those skilled in the art that various changes in form and detail thereof may be made without departing from the scope of the claimed invention.

## Claims

1. A method of making a tissue engineering device for use in the repair or regeneration of tissue comprising:
providing a support scaffold layer;
culturing a cell population on a non-adherent substrate to promote extracelullar matrix protein production to produce a cell sheet layer comprising extracellular matrix protein;
removing the cell sheet layer from the non-adherent substrate; and
disposing the cell sheet layer on the support scaffold layer,
provided that the method does not comprise the use of human embryonic stem cells.

2. The method of claim 1 wherein said cell sheet layer is comprised of chondrocytes, osteoblasts, fibroblasts, angioblasts, myoblasts, epithelial cells, urothelial cells, smooth muscle cells, keratinocytes, beta cells, endothelial cells, fibrocytes, vascular endothelial cells, hepatocytes, small intestine epithelial cells, epidermal keratinocytes, bone marrow mesenchymal cells, cardiomyocytes, intervertebral disc cells, oral mucosal epithelial cells, gastrointestinal mucosal epithelial cells, urinary tract epithelial cells, skeletal joint synovium cells, periosteum cells, perichondrium cells, skeletal muscle cells, smooth muscle cells, cardiac muscle cells, pericardium cells, dural cells, meningeal cells, keratinocyle-precursor cells, pericytes, glial cells, neural cells, amniotic membrane cells, placental membrane cells, serosal cells, undifferentiated stem cells, undifferentiated progenitor cells, pre-differentiated stem cells, pre-differentiated progenitor cells, neural stem cells, neural progenitor cells, neuronal cells, dendritic cells or genetically engineered cells.

3. The method of claim 1 wherein said cell sheet layer is comprised of stem cells.

4. The method of claim 3 wherein said stem cells are hematopoietic, mesenchymal, postpartum, pancreatic, hepatic, retinal epithelial, olfactory bulb, endothelial, muscle, adipose-derived, ilcac crest, bone marrow, periodontal ligament, oval or dermal cells or organ specific stem or progenitor cells.

5. The method of claim 1 wherein said cell sheet layer further comprises a growth factor.

6. The method of claim 5, wherein said growth factor is vascular endothelial cell growth factor, platelet derived growth factor, epidermal growth factor, fibroblast growth factor, hepatocyte growth factor, insulin-like growth factor, brain derived neurotrophic factor, growth differentiation factor-5, EPO or transforming growth factor.

7. The method of claim 1 wherein said cell sheet layer further comprises a reinforcing member.

8. The method of claim 1 wherein said support scaffold layer comprises a biodegradable homopolymer, copolymer or polymer blend comprised of lactide, glycolide, epsilon-caprolactone, 1,4-dioxan-2-one, 1,3-dioxan-2-one or alkyl derivative of trimethylene carbonate monomers.

9. The method of claim 1 wherein said support scaffold layer comprises a fibrin-based material, collagen-based material, hyaluronic acid-based material, glycoprotein-based material, celluose-based material or silk.

## Patentansprüche

1. Verfahren zum Herstellen einer Vorrichtung zur Gewebezüchtung (*Tissue engineering*) zur Verwendung zur Reparatur oder Regeneration von Gewebe, das Folgendes umfasst:
Verfügbarmachen einer Trägergerüst-Schicht;
Kultivieren einer Zellpopulation an einem nichtadhärenten Substrat, um eine Herstellung von extrazellulärem Matrixprotein zu fördern, um eine Zellsheet-Schicht herzustellen, die extrazelluläres Matrixprotein umfasst;
Entfernen der Zellsheet-Schicht von dem nichtadhärenten Substrat; und
Anordnen der Zellsheet-Schicht an der Trägergerüst-Schicht, vorausgesetzt, dass das Verfahren keine Verwendung von menschlichen embryonalen Stammzellen umfasst.

2. Verfahren nach Anspruch 1, wobei die Zellsheet-Schicht Chondrozyten, Osteoblasten, Fibroblasten, Angioblasten, Myoblasten, Epithelzellen, Urothelzellen, glatte Muskelzellen, Keratinozyten, Betazellen, Endothelzellen, Fibrozyten, Gefäßendothelzellen, Hepatozyten, Dünndarmeptithelzellen, epidermale Keratinozyten, Mesenchymzellen des Knochenmarks, Kardiomyozyten, Bandscheibenzellen, Epithelzellen der oralen Mucosa, Epithelzellen der gastrointestinalen Mucosa, Epithelzellen der Harnwege, Zellen der Skelettgelenksynovialmembran, Periostzellen, Zellen des Perichondriums, Skelettmuskelzellen, glatte Muskelzellen, Herzmuskelzellen, Perikardzellen, Zellen der Dura mater, Meningealzellen, Keratinozyten-Vorläuferzellen, Perizyten, Gliazellen, Nervenzellen, Amnionmembranzellen, Plazentamembranzellen, Serosazellen, undifferenzierte Stammzellen, undifferenzierte Progenitorzellen, vordifferenzierte Stammzellen, vordifferenzierte Progenitorzellen, neurale Stammzellen, neurale Progenitorzellen, neuronale Zellen, dendritische Zellen oder gentechnisch erzeugte Zellen umfasst.

3. Verfahren nach Anspruch 1, wobei die Zellsheet-Schicht Stammzellen umfasst.

4. Verfahren nach Anspruch 3, wobei die Stammzellen hämatopoetische, Mesenchym-, Postpartum-, Pankreas-, hepatische, Retinaepithel-, Riechkolben-, Endothel-, Muskel-, von Körperfett abgeleitete, Beckenkamm-, Knochenmark-, Peridontalligament-, Oval- oder Dermalzellen oder organspezifische Stamm- oder Progenitorzellen sind.

5. Verfahren nach Anspruch 1, wobei die Zellsheet-Schicht weiterhin einen Wachstumsfaktor umfasst.

6. Verfahren nach Anspruch 5, wobei der Wachstumsfaktor ein Gefäßendothelzellwachstumsfaktor, *Platelet derived growth factor,* Epidermiswachstumsfaktor, Fibrobastenwachstumsfaktor, Hepatozytenwachstumsfaktor, Insulin-artiger Wachstumsfaktor, *Brain derived neurotrophic factor,* Wachstumsdifferenzierungsfaktor-5, EPO oder transformierender Wachstumsfaktor ist.

7. Verfahren nach Anspruch 1, wobei die Zellsheet-Schicht weiterhin ein verstärkendes Element umfasst.

8. Verfahren nach Anspruch 1, wobei die Trägergerüst-Schicht ein biologisch abbaubares Homopolymer, Copolymer oder eine Polymermischung, die Laktid, Glykolid, Epsilon-Caprolakton, 1,4-Dioxan-2-on, 1,3-Dioxan-2-on oder ein Alkylderivat von Trimethylencarbonat-Monomeren umfasst, umfasst.

9. Verfahren nach Anspruch 1, wobei die Trägergerüst-Schicht ein fibrinbasiertes Material, kollagenbasiertes Material, hyaluronsäurebasiertes Material, glycoproteinbasiertes, zellulosebasiertes Material oder Seide umfasst.

## Revendications

1. Méthode de réalisation d'un dispositif d'ingénierie tissulaire à utiliser dans la réparation ou la régénération de tissu, comprenant :
➢ la fourniture d'une couche de support ;
➢ la culture d'une population cellulaire sur un substrat non-adhérent pour promouvoir la production de protéine matrice extracellulaire, afin de produire une couche de feuille cellulaire comprenant une protéine de matrice extracellulaire ;
➢ l'élimination de la couche de feuille cellulaire du substrat non-adhérent, et
➢ la disposition de la couche de feuille cellulaire sur la couche de support,
sachant que la méthode ne comprenne pas l'utilisation de cellules souches d'embryons humains.

2. Méthode selon la revendication 1, dans laquelle ladite couche de feuille cellulaire comprend des chondrocytes, des ostéoblastes, des fibroblastes, des angioblastes, des myoblastes, des cellules épithéliales, des cellules urothéliales, des cellules de muscle lisse, des kératinocytes, des cellules bêta, des cellules endothéliales, des fibrocytes, des cellules endothéliales vasculaires, des hépatocytes, des cellules épithéliales de l'intestin grêle, des kératinocytes épidermiques, des cellules mésenchymateuses de la moelle osseuse, des cardiomyocytes, des cellules de disque intervertébral, des cellules épithéliales de la muqueuse orale, des cellules épithéliales des muqueuses gastro-intestinales, des cellules épithéliales des voies urinaires, des cellules synoviales de l'articulation squelettique, des cellules périostiques, des cellules du périchondre, des cellules du muscle squelettique, des cellules du muscle lisse, des cellules du muscle cardiaque, des cellules du péricarde, des cellules durales, des cellules méningées, des cellules de précurseur de kératinocytes, des péricytes, des cellules gliales, des cellules neurales, des cellules de membrane amniotique, des cellules de membrane placentaire, des cellules séreuses, des cellules souches indifférenciées, des progéniteurs indifférenciés, des cellules souches prédifférenciées, des progéniteurs prédifférenciés, des cellules souches neurales, des progéniteurs neuraux, des cellules neuronales, des cellules dendritiques ou des cellules génétiquement modifiées.

3. Méthode selon la revendication 1, dans laquelle ladite couche de feuille cellulaire comprend des cellules souches.

4. Méthode selon la revendication 3, dans laquelle lesdites cellules souches sont des cellules hématopoïétiques, mésenchymateuses, postpartum, pancréatiques, hépatiques, épithéliales rétiniennes, du bulbe olfactif, endothéliales, musculaires, dérivées de l'adipose, de la crête iliaque, de moelle osseuse, du ligament parodontal, ovariennes ou dermiques ou des cellules souches ou progéniteurs spécifiques à un organe.

5. Méthode selon la revendication 1, dans laquelle ladite couche de feuille cellulaire comprend en outre un facteur de croissance.

6. Méthode selon la revendication 5, dans laquelle ledit facteur de croissance est un facteur de croissance de cellule endothéliale vasculaire, un facteur de croissance dérivé des plaquettes, un facteur de croissance épidermique, un facteur de croissance de fibroblaste, un facteur de croissance d'hépatocyte, un facteur de croissance semblable à l'insuline, un facteur neurotrophique dérivé du cerveau, un facteur de différenciation de croissance-5, l'EPO ou un facteur de croissance de transformation.

7. Méthode selon la revendication 1, dans laquelle ladite couche de feuille cellulaire comprend en outre un élément de renfort.

8. Méthode selon la revendication 1, dans laquelle ladite couche de support comprend un homopolymère, copolymère ou mélange de polymère biodégradable comprenant un lactide, un glycolide, une epsilon-caprolactone, une 1,4-dioxan-2-one, une 1,3-dioxan-2-one, ou un dérivé alkyle de monomères de triméthylène carbonate.

9. Méthode selon la revendication 1, dans laquelle ladite couche de support comprend un matériau à base de fibrine, un matériau à base de collagène, un matériau à base d'acide hyaluronique, un matériau à base de glycoprotéine, un matériau à base de cellulose ou de la soie.
